# EUROPEAN PATENT APPLICATION

(11) **EP 4 620 462 A1**
(43) Date of publication of application: **24.09.2025**
(21) Application number: 24165691.7
(22) Date of filing: 22.03.2024
(51) Int. Cl.: A61K 31/00, A61N 5/00, A61P 25/22, A61P 25/24

(54) **MOOD STATE MODULATION**

(71) Applicant: Nicoventures Trading Limited, London WC2R 3LA (GB)
(72) Inventor: SMITH, Jeffrey, Winston-Salem, 27102 (US); AZZOPARDI, Anna, London, WC2R 3LA (GB); SOKOLI, Hava, London, WC2R 3LA (GB)
(74) Representative: D Young & Co LLP

(57) **Abstract**

There is provided a method of modulating a mood state of a subject comprising administering one or more cannabinoids in combination with the application of an electrical neurological stimulus to the subject, wherein the electrical neurological stimulus is millimetre wave technology.

## Description

### Field

The present specification relates to modulation of a mood state of a subject.

### Background

The wellness industry is growing, with consumers increasingly interested in their overall physical and mental wellbeing and/or performance. Therefore, methods and products that can provide a beneficial effect such as enhancing the wellbeing of an individual, be that through better sleep, increased mindfulness or other measures of 'wellness', have become popular and are of increased commercial importance. Such products may include bioactive ingredients that are delivered to the user in order to cause a biological response in the user that may enhance physical or mental wellbeing and/or performance of the user.

Therefore, it would be desirable to provide consumers with non-therapeutic products to modulate mood, enhance performance and/or provide other benefits such as providing an anti-fatigue effect, restoring balance and increasing adaptability to overcome daily physical and mental challenges. Whilst methods and systems are known for delivering one or more active compounds to a subject that seek to change or control a mood state of the subject, there remains a need for further developments in this field.

### Summary

Disclosed herein is a method of modulating a mood state of a subject comprising administering one or more cannabinoids in combination with the application of an electrical neurological stimulus to the subject, wherein the electrical neurological stimulus is millimetre wave technology.

In one embodiment, the one or more cannabinoids are inhaled. For example, in one embodiment, the one or more cannabinoids are administered by an aerosol delivery device.

In one embodiment, the one or more cannabinoids are ingested. For example, in one embodiment, the one or more cannabinoids are ingested orally. For example, in one embodiment, the one or more cannabinoids are administered in edible form.

In one embodiment, the one or more cannabinoids are administered at a total daily dose of no greater than 200 mg.

In one embodiment, the one or more cannabinoids are administered twice a day, once as a first dose and once as a second dose, wherein the first dose and the second dose are each independently administered in an amount from 5 and 50 mg.

In one embodiment, the one or more cannabinoids have an initial point of administration that is concurrent with, before or after the application of the electrical neurological stimulus.

In one embodiment, a period of time between the initial point of administration of the one or more cannabinoids and applying the electrical neurological stimulus is no greater than 60 minutes.

In one embodiment, the electrical neurological stimulus is applied by an implant.

In one embodiment, the electrical neurological stimulus is applied by a wearable device.

In one embodiment, the wearable device is a bracelet, a watch, a headband, or chestband.

In one embodiment, the wearable device is a bracelet.

In one embodiment, the electrical neurological stimulus is applied twice a day.

In one embodiment, the one or more cannabinoids are administered twice a day, once as a first dose and once as a second dose and the electrical neurological stimulus is applied twice a day, once as a first application and once as a second application.

In one embodiment, the mood state comprises the subject's ability to concentrate.

In one embodiment, the mood state comprises the subject's perceived sleepiness.

In another aspect, provided herein is a non-therapeutic method of modulating a mood state of a subject comprising administering one or more cannabinoids in combination with the application of an electrical neurological stimulus to the subject, wherein the electrical neurological stimulus is millimetre wave technology.

In a further aspect, provided herein is a system comprising: one or more cannabinoids for delivery to a user in order to modulate a mood state of said user; and a delivery mechanism for applying an electrical neurological stimulus to a user in order to modulate a mood state of said user, wherein the electrical neurological stimulus is millimetre wave technology.

In one embodiment, the system further comprises a control mechanism that controls the delivery of the one or more cannabinoids and the application of the electrical neurological stimulus to the user.

### Brief Description of the Drawings

Example embodiments will now be described, by way of example only, with reference to the following figures, in which:
Figure 1 shows the results of a POMS survey (ability to concentrate measure) over a 5 week active trial as described in Example 1.
Figure 2 shows the results of an RSQ (perceived sleepiness measure) over a 5 week active trial described as in Example 1.

### Detailed Description

This disclosure is not limited by the exemplary methods and materials disclosed herein, and any methods and materials similar or equivalent to those described herein can be used in the practice or testing of embodiments of this disclosure.

The headings provided herein are not limitations of the various aspects or embodiments of this disclosure which can rather be had by reference to the specification as a whole. Accordingly, the terms defined immediately below are more fully defined by reference to the specification as a whole and combinations of the features disclosed herein are expressly provided for.

It must be noted that as used herein and in the appended claims, the singular forms "a", "an", and "the" include plural referents unless the context clearly dictates otherwise. Thus, for example, reference to "a bioactive substance" or "a flavour" includes a plurality of such candidate agents and equivalents thereof known to those skilled in the art, and so forth.

In a first aspect, the invention provides a method of modulating a mood state of a subject comprising administering one or more cannabinoids in combination with the application of an electrical neurological stimulus to the subject, wherein the electrical neurological stimulus is millimetre wave technology.

In this regard, the present inventors have surprisingly found that administering one or more cannabinoids in combination with the application of an electrical neurological stimulus in the form of millimetre wave technology can provide a beneficial impact upon the mood state of a subject. For example, a positive impact on at least a subject's ability to concentrate and perceived sleepiness can be provided.

### Subject

As used herein, "subject" refers to the recipient of the method disclosed herein. The "subject" may be human or animal. In some embodiments, the subject is human. In some embodiments, the subject is an animal. Where the subject is an animal, the animal may be mammalian, avian, reptilian, amphibian, invertebrate or aquatic (fish). Where the animal is a mammalian animal, the mammalian animal may be a monotreme (such as a platypus or echidna), marsupial (such as a kangaroo, wallaby, wambat, etc.), or a placental mammal (such as a primate - monkeys, apes; canine - dogs, wolves, foxes, etc.; feline - domestic cats, lions, tigers, etc.).

In some embodiments, the subject is a healthy individual. For example, the subject has not been diagnosed with any disease, medical condition, or deficiency.

### Mood state

A mood state of a subject may depend on a number of environmental factors such as work, exercise, gaming or any activity. Example embodiments described herein relate to the control of delivery of active compounds and electrical neurological stimuli for the purpose of managing, controlling or influencing mood states.

In some embodiments, the mood state of the subject comprises the subject's ability to concentrate. In some embodiments, the method of the invention provides for an increase in the subject's ability to concentrate. In some embodiments, the mood state of the subject relates to the subject's perceived sleepiness. In some embodiments, the method of the invention provides for a decrease in the subject's perceived sleepiness. In some embodiments, the method of the invention provides for a decrease in the subject's perceived sleepiness upon waking.

### Cannabinoids

As disclosed herein the method of the present invention comprises the administration of one or more cannabinoids.

Cannabinoids are a class of natural or synthetic chemical compounds which act on, for example, cannabinoid receptors (i.e., CB1 and CB2) in cells that repress neurotransmitter release in the brain. Cannabinoids are cyclic molecules exhibiting particular properties such as the ability to easily cross the blood-brain barrier. Cannabinoids may be naturally occurring (Phytocannabinoids) from plants such as cannabis, (endocannabinoids) from animals, or artificially manufactured (synthetic cannabinoids). Cannabis species express at least 85 different phytocannabinoids, and these may be divided into subclasses, including cannabigerols, cannabichromenes, cannabidiols, tetrahydrocannabinols, cannabinols and cannabinodiols, and other cannabinoids.

According to one embodiment, the at least one cannabinoid may be selected from cannabigerol (CBG), cannabigerolic acid (CBGA), cannabichromene (CBC), cannabichromenic acid (CBCA), cannabidiol (CBD), cannabidolic acid (CBDA), tetrahydrocannabinol (THC), including its isomers Δ^{6a,10a}-tetrahydrocannabinol (Δ^{6a,10a}-THC), Δ^{6a(7)}-tetrahydrocannabinol (Δ^{6a(7)}-THC), Δ⁸-tetrahydrocannabinol (Δ⁸-THC), Δ⁹-tetrahydrocannabinol (Δ⁹-THC), Δ¹⁰-tetrahydrocannabinol (Δ¹⁰-THC), Δ^{9,11}-tetrahydrocannabinol (Δ^{9,11}-THC), tetrahydrocannabinolic acid (THCA),cannabinol (CBN), cannabinolic acid (CBNA), and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM) and combinations thereof.

In one embodiment the cannabinoid(s) of interest are selected from cannabidiol (CBD), Δ⁸-tetrahydrocannabinol (Δ⁸-THC), Δ⁹-tetrahydrocannabinol (Δ⁹-THC). In one embodiment the cannabinoid of interest is cannabidiol (CBD). In one embodiment the cannabinoid of interest is Δ⁸-tetrahydrocannabinol (Δ⁸-THC). In one embodiment the cannabinoid of interest is Δ⁹-tetrahydrocannabinol (Δ⁹-THC). In one embodiment the cannabinoid of interest is cannabinol (CBN).

In one embodiment the cannabinoid(s) of interest are selected from cannabidiol (CBD), Δ⁸-tetrahydrocannabinol (Δ⁸-THC), Δ⁹-tetrahydrocannabinol (Δ⁹-THC), cannabinol (CBN), cannabigerol (CBG), cannabichromene (CBC), and mixtures thereof. In one embodiment the cannabinoid of interest is at least cannabidiol (CBD). In one embodiment the cannabinoid of interest is at least Δ⁸-tetrahydrocannabinol (Δ⁸-THC). In one embodiment the cannabinoid of interest is at least Δ⁹-tetrahydrocannabinol (Δ⁹-THC). In one embodiment the cannabinoid of interest is at least cannabinol (CBN). In one embodiment the cannabinoid of interest is at least cannabigerol (CBG). In one embodiment the cannabinoid of interest is at least cannabichromene (CBC), and mixtures thereof.

Naturally derived cannabinoids are generally present in their carboxylated form. In this regard, cannabidiol (CBD) and cannabidolic acid (CBDA) are the respective decarboxylated and carboxylated forms.

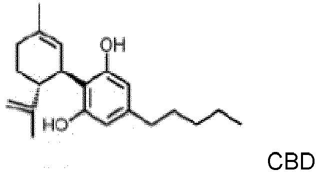

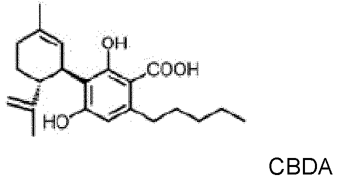

The cannabinoid referred to herein may be present in either the carboxylated or decarboxylated form. Exemplary carboxylated cannabinoid in this regard include cannabigerolic acid (CBGA), cannabichromenic acid (CBCA), cannabinolic acid (CBNA), tetrahydrocannabinolic acid (THCA), cannabidolic acid (CBDA) and combinations thereof.

In one embodiment, the cannabinoid is preferably cannabidiol (CBD) or a pharmaceutically acceptable salt thereof. In one embodiment, the cannabidiol is synthetic cannabidiol. In one embodiment, the cannabidiol is provided in the form of an extract or distillate.

In an embodiment, a delivery system for delivering the one or more cannabinoids is an aerosol-free delivery system.

In an embodiment, the delivery system is an aerosol-free delivery system that delivers at least one substance to a user orally, nasally, transdermally or in another way without forming an aerosol, including but not limited to, lozenges, gums, and oral products such as snus or moist snuff. Whilst snus or moist snuff are traditionally tobacco products, the contents of the snus pouch or snuff may be something other than tobacco.

In one embodiment, the one or more cannabinoids are ingested. In one embodiment, the one or more cannabinoids are ingested orally. In a further embodiment, the aerosol-free delivery system is an edible, a lozenge, a gum, a beverage, a shot, an oil, an ointment, a supplement or an oral product. In one embodiment the aerosol-free delivery system is an edible. In one embodiment, the one or more cannabinoids is a gummy.

In one embodiment, the one or more cannabinoids are inhaled. For example, in one embodiment, the one or more cannabinoids are administered by an aerosol delivery device.

In one embodiment, the aerosol delivery device is a non-combustible aerosol provision system that releases compounds from an aerosolisable material without combusting the aerosolisable material, such as electronic vaporisers and hybrid systems to generate aerosol using a combination of aerosolisable materials.

According to the present disclosure, a "non-combustible" aerosol provision system is one where a constituent aerosol-generating material of the aerosol provision system (or component thereof) is not combusted or burned in order to facilitate delivery of at least one substance to a user.

In some embodiments, the delivery system is a non-combustible aerosol provision system, such as a powered non-combustible aerosol provision system.

In some embodiments, the non-combustible aerosol provision system is a vaping device or electronic nicotine delivery system (END), although it is noted that the presence of nicotine in the aerosol-generating material is not a requirement. In some embodiments, the non-combustible aerosol provision system is an aerosol-generating material heating system, also known as a heat-not-burn system.

In some embodiments, the non-combustible aerosol provision system is a hybrid system to generate aerosol using a combination of aerosol-generating materials, one or a plurality of which may be heated. Each of the aerosol-generating materials may be, for example, in the form of a solid, liquid or gel and may or may not contain nicotine. In some embodiments, the hybrid system comprises a liquid or gel aerosol-generating material and a solid aerosol-generating material.

Typically, the non-combustible aerosol provision system may comprise a non-combustible aerosol provision device and a consumable for use with the non-combustible aerosol provision device.

In some embodiments, the disclosure relates to consumables comprising an aerosol-generating material optionally comprising the one or more cannabinoids and configured to be used with non-combustible aerosol provision devices. These consumables are sometimes referred to as articles throughout the disclosure.

In some embodiments, the non-combustible aerosol provision system, such as a non-combustible aerosol provision device thereof, may comprise a power source and a controller. The power source may, for example, be an electric power source or an exothermic power source. In some embodiments, the exothermic power source comprises a carbon substrate which may be energised so as to distribute power in the form of heat to an aerosol-generating material or to a heat transfer material in proximity to the exothermic power source.

In some embodiments, the non-combustible aerosol provision system may comprise an area for receiving the consumable, an aerosol generator, an aerosol generation area, a housing, a mouthpiece, a filter and/or an aerosol-modifying agent.

In some embodiments, the consumable for use with the non-combustible aerosol provision device may comprise aerosol-generating material, an aerosol-generating material storage area, an aerosol-generating material transfer component, an aerosol generator, an aerosol generation area, a housing, a wrapper, a filter, a mouthpiece, and/or an aerosol-modifying agent.

In some embodiments, the substance to be delivered may be an aerosol-generating material or a material that is not intended to be aerosolised, wherein the material optionally comprises the one or more cannabinoids. As appropriate, either material may comprise one or more active constituents, one or more flavours, one or more aerosol-former materials, and/or one or more other functional materials.

Aerosolisable material, which also may be referred to herein as aerosol generating material, is material that is capable of generating aerosol, for example when heated, irradiated or energized in any other way. Aerosolisable material may, for example, be in the form of a solid, liquid or gel which may or may not contain flavourants.

The aerosol-generating material may be an "amorphous solid". In some embodiments, the amorphous solid is a "monolithic solid". The aerosol-generating material may be non-fibrous or fibrous. In some embodiments, the aerosol-generating material may be a dried gel. The aerosol-generating material may be a solid material that may retain some fluid, such as liquid, within it. In some embodiments the retained fluid may be water (such as water absorbed from the surroundings of the aerosol-generating material) or the retained fluid may be solvent (such as when the aerosol-generating material is formed from a slurry). In some embodiments, the solvent may be water.

The aerosol-generating material may comprise one or more active substances and/or flavours, one or more aerosol-former materials, and optionally one or more other functional materials. The aerosol-former material may comprise one or more constituents capable of forming an aerosol. In some embodiments, the aerosol-former material may comprise one or more of glycerine, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

The one or more other functional materials may comprise one or more of pH regulators, colouring agents, preservatives, binders, fillers, stabilizers, and/or antioxidants.

The material may be present on or in a support, to form a substrate. The support may, for example, be or comprise paper, card, paperboard, cardboard, reconstituted material, a plastics material, a ceramic material, a composite material, glass, a metal, or a metal alloy. In some embodiments, the support comprises a susceptor. In some embodiments, the susceptor is embedded within the material. In some alternative embodiments, the susceptor is on one or either side of the material. In some alternative embodiments, the susceptor surrounds the material.

A consumable is an article comprising or consisting of aerosol-generating material, part or all of which is intended to be consumed during use by a user. A consumable may comprise one or more other components, such as an aerosol-generating material storage area, an aerosol-generating material transfer component, an aerosol generation area, a housing, a wrapper, a mouthpiece, a filter and/or an aerosol-modifying agent. A consumable may also comprise an aerosol generator, such as a heater, that emits heat to cause the aerosol-generating material to generate aerosol in use. The heater may, for example, comprise a material heatable by electrical conduction, or a susceptor.

An aerosol generator is an apparatus configured to cause aerosol to be generated from the aerosol-generating material. In some embodiments, the aerosol generator is a heater configured to subject the aerosol-generating material to heat energy, so as to release one or more volatiles from the aerosol-generating material to form an aerosol. In some embodiments, the aerosol generator is configured to cause an aerosol to be generated from the aerosol-generating material without heating. For example, the aerosol generator may be configured to subject the aerosol-generating material to one or more of vibration, increased pressure, or electrostatic energy.

In one embodiment, the one or more cannabinoids are administered once a day. In one embodiment, the one or more cannabinoids are administered twice a day. In one embodiment, the one or more cannabinoids are administered three times a day. In one embodiment, the one or more cannabinoids are administered four times a day. In one embodiment, the one or more cannabinoids are administered five times a day.

In one embodiment, the one or more cannabinoids are administered twice a day, once as a first dose and once as a second dose. In one embodiment, the first dose is administered in the morning and the second dose is administered in the evening. In one embodiment, the morning is defined as being between the hours of five am and noon. In one embodiment, the evening is defined as being between the hours of four pm and eleven pm.

In one embodiment, the one or more cannabinoids are administered at a maximum daily dose of no greater than 600 mg. In one embodiment, the one or more cannabinoids are administered at a maximum daily dose of no greater than 550 mg. In one embodiment, the one or more cannabinoids are administered at a maximum daily dose of no greater than 500 mg. In one embodiment, the one or more cannabinoids are administered at a maximum daily dose of no greater than 450 mg. In one embodiment, the one or more cannabinoids are administered at a maximum daily dose of no greater than 400 mg. In one embodiment, the one or more cannabinoids are administered at a maximum daily dose of no greater than 350 mg. In one embodiment, the one or more cannabinoids are administered at a maximum daily dose of no greater than 300 mg. In one embodiment, the one or more cannabinoids are administered at a maximum daily dose of no greater than 250 mg. In one embodiment, the one or more cannabinoids are administered at a maximum daily dose of no greater than 200 mg. In one embodiment, the one or more cannabinoids are administered at a maximum daily dose of no greater than 150 mg. In one embodiment, the one or more cannabinoids are administered at a maximum daily dose of no greater than 100 mg. In one embodiment, the one or more cannabinoids are administered at a maximum daily dose of no greater than 75 mg. In one embodiment, the one or more cannabinoids are administered at a maximum daily dose of no greater than 60 mg. In one embodiment, the one or more cannabinoids are administered at a maximum daily dose of no greater than 50 mg. In one embodiment, the one or more cannabinoids are administered at a maximum daily dose of no greater than 25 mg.

In one embodiment, the one or more cannabinoids are administered at a maximum daily dose of at least 1 mg. In one embodiment, the one or more cannabinoids are administered at a maximum daily dose of at least 5 mg. In one embodiment, the one or more cannabinoids are administered at a maximum daily dose of at least 10 mg. In one embodiment, the one or more cannabinoids are administered at a maximum daily dose of at least 15 mg. In one embodiment, the one or more cannabinoids are administered at a maximum daily dose of at least 20 mg. In one embodiment, the one or more cannabinoids are administered at a maximum daily dose of at least 25 mg. In one embodiment, the one or more cannabinoids are administered at a maximum daily dose of at least 30 mg. In one embodiment, the one or more cannabinoids are administered at a maximum daily dose of at least 35 mg. In one embodiment, the one or more cannabinoids are administered at a maximum daily dose of at least 40 mg. In one embodiment, the one or more cannabinoids are administered at a maximum daily dose of at least 45 mg. In one embodiment, the one or more cannabinoids are administered at a maximum daily dose of at least 50 mg. In one embodiment, the one or more cannabinoids are administered at a maximum daily dose of at least 100 mg.

In one embodiment, the one or more cannabinoids are administered at a maximum daily dose of from 5 mg to 600 mg. In one embodiment, the one or more cannabinoids are administered at a maximum daily dose of from 5 mg to 550 mg. In one embodiment, the one or more cannabinoids are administered at a maximum daily dose of from 5 mg to 500 mg. In one embodiment, the one or more cannabinoids are administered at a maximum daily dose of from 5 mg to 450 mg. In one embodiment, the one or more cannabinoids are administered at a maximum daily dose of from 5 mg to 400 mg. In one embodiment, the one or more cannabinoids are administered at a maximum daily dose of from 5 mg to 350 mg. In one embodiment, the one or more cannabinoids are administered at a maximum daily dose of from 5 mg to 300 mg. In one embodiment, the one or more cannabinoids are administered at a maximum daily dose of from 5 mg to 250 mg. In one embodiment, the one or more cannabinoids are administered at a maximum daily dose of from 5 mg to 200 mg. In one embodiment, the one or more cannabinoids are administered at a maximum daily dose of from 5 mg to 150 mg. In one embodiment, the one or more cannabinoids are administered at a maximum daily dose of from 5 mg to 100 mg. In one embodiment, the one or more cannabinoids are administered at a maximum daily dose of from 5 mg to 80 mg. In one embodiment, the one or more cannabinoids are administered at a maximum daily dose of from 5 mg to 70 mg. In one embodiment, the one or more cannabinoids are administered at a maximum daily dose of from 5 mg to 60 mg. In one embodiment, the one or more cannabinoids are administered at a maximum daily dose of from 5 mg to 50 mg.

In one embodiment, the one or more cannabinoids are administered at a maximum daily dose of from 10 mg to 100 mg. In one embodiment, the one or more cannabinoids are administered at a maximum daily dose of from 10 mg to 80 mg. In one embodiment, the one or more cannabinoids are administered at a maximum daily dose of from 20 mg to 100 mg. In one embodiment, the one or more cannabinoids are administered at a maximum daily dose of from 20 mg to 80 mg.

In one embodiment, each individual dose of the one or more cannabinoids is from 1-60 mg. In one embodiment, each individual dose of the one or more cannabinoids is from 1-50 mg. In one embodiment, each individual dose of the one or more cannabinoids is from 1 - 40 mg. In one embodiment, each individual dose of the one or more cannabinoids is from 1-30 mg. In one embodiment, each individual dose of the one or more cannabinoids is from 1-20 mg. In one embodiment, each individual dose of the one or more cannabinoids is from 1-10 mg. In one embodiment, each individual dose of the one or more cannabinoids is from of 1 - 5 mg.

In one embodiment, each individual dose of the one or more cannabinoids is from 5-60 mg. In one embodiment, each individual dose of the one or more cannabinoids is from 10 -60 mg. In one embodiment, each individual dose of the one or more cannabinoids is from 20 - 60 mg. In one embodiment, each individual dose of the one or more cannabinoids is from 30 - 60 mg. In one embodiment, each individual dose of the one or more cannabinoids is from 40 - 60 mg. In one embodiment, each individual dose of the one or more cannabinoids is from 50 - 60 mg.

In one embodiment, each individual dose of the one or more cannabinoids is about 5 mg. In one embodiment, each individual dose of the one or more cannabinoids is about 10 mg. In one embodiment, each individual dose of the one or more cannabinoids is about 15 mg. In one embodiment, each individual dose of the one or more cannabinoids is about 20 mg. In one embodiment, each individual dose of the one or more cannabinoids is about 25 mg. In one embodiment, each individual dose of the one or more cannabinoids is about 30 mg. In one embodiment, each individual dose of the one or more cannabinoids is about 35 mg. In one embodiment, each individual dose of the one or more cannabinoids is about 40 mg. In one embodiment, each individual dose of the one or more cannabinoids is about 45 mg. In one embodiment, each individual dose of the one or more cannabinoids is about 50 mg. In one embodiment, each individual dose of the one or more cannabinoids is about 55 mg. In one embodiment, each individual dose of the one or more cannabinoids is about 60 mg.

In one embodiment, the one or more cannabinoids are administered twice a day, once as a first dose and once as a second dose, wherein the first dose and second dose are independently administered in an amount of from 5 to 50 mg. In one embodiment, the first dose is about 30 mg and the second dose is about 30 mg. In one embodiment, the one or more cannabinoids are administered twice a day as a gummy, once as a first dose and once as a second dose, wherein the first dose and second dose are independently administered in an amount of from 5 to 50 mg. In one embodiment, the one or more cannabinoids are administered twice a day as a gummy, once as a first dose and once as a second dose, wherein the first dose and second dose are independently administered in an amount of about 30 mg.

### Electrical neurological stimulus

As used herein, electrical neurological stimulus relates to the stimulation of a subject's nervous system in order to disrupt electrical pathways therein. The stimulus can be provided via, for example wearable and implantable electrodes and devices.

The term covers a broad range of stimuli such as electrical muscle stimulation (EMS), Neuromuscular electrical stimulation (NMES), Functional electrical stimulation (FES), Transcutaneous electrical nerve stimulation (TENS), vagus nerve stimulation and transcranial direct current stimulation (tDCS). Generally speaking, the stimulation techniques reflect either the intended use of the electrical stimulation or the characteristics of the stimulation itself. For example, EMS typically uses lower-frequency rectangular waveforms.

One particular area of interest relates to brain stimulation techniques. These techniques act by activating or inhibiting the brain with electricity. The electricity can be given directly through electrodes implanted in the brain or indirectly through electrodes placed on the scalp. The electricity can also be induced by applying magnetic fields to the head.

As disclosed herein, the electrical neurological stimulus comprises millimetre wave technology. Millimetre waves are a form of microwaves and are non-ionizing. These waves are typically administered onto a localized area of the skin at a sufficiently low intensity that there is no perceptible heating.

The present invention is directed towards the use of millimetre wave technology in combination with one or more cannabinoids for modulating a mood state of a subject.

In one embodiment, the electrical neurological stimulus is applied by an implant.

In one embodiment, the electrical neurological stimulus is applied by a wearable device. In one embodiment, the wearable device is a bracelet. In one embodiment, the wearable device is a watch. In one embodiment, the wearable device is a headband. In one embodiment, the wearable device is a chestband. In one embodiment, the electrical neurological stimulus is applied by a combination of a bracelet, a watch, a headband, and/or a chestband.

In one embodiment, the electrical neurological stimulus is applied once a day. In one embodiment, the electrical neurological stimulus is applied twice a day. In one embodiment, the electrical neurological stimulus is applied three times a day. In one embodiment, the electrical neurological stimulus is applied four times a day. In one embodiment, the electrical neurological stimulus is applied five times a day.

In one embodiment, the electrical neurological stimulus is applied for the same number of times a day as the one or more cannabinoids are administered. In one embodiment, the one or more cannabinoids are administered twice a day, once as a first dose and once as a second dose and the electrical neurological stimulus is applied twice a day, once as a first application and once as a second application.

In one embodiment, the one or more cannabinoids have an initial point of administration that is concurrent with the electrical neurological stimulus. In one embodiment, the one or more cannabinoids have an initial point of administration that is before the electrical neurological stimulus. In one embodiment, the one or more cannabinoids have an initial point of administration that is after the electrical neurological stimulus.

In one embodiment, the electrical neurological stimulus is applied at a frequency of from 30 to 300 GHz. In one embodiment, the electrical neurological stimulus is applied at a frequency of from 30 to 250 GHz. In one embodiment, the electrical neurological stimulus is applied at a frequency of from 30 to 200 GHz. In one embodiment, the electrical neurological stimulus is applied at a frequency of from 30 to 150 GHz. In one embodiment, the electrical neurological stimulus is applied at a frequency of from 30 to 100 GHz. In one embodiment, the electrical neurological stimulus is applied at a frequency of from 30 to 90 GHz. In one embodiment, the electrical neurological stimulus is applied at a frequency of from 30 to 80 GHz. In one embodiment, the electrical neurological stimulus is applied at a frequency of from 30 to 70 GHz. In one embodiment, the electrical neurological stimulus is applied at a frequency of from 30 to 65 GHz. In one embodiment, the electrical neurological stimulus is applied at a frequency of from 30 to 60 GHz. In one embodiment, the electrical neurological stimulus is applied at a frequency of from 30 to 55 GHz. In one embodiment, the electrical neurological stimulus is applied at a frequency of from 30 to 50 GHz. In one embodiment, the electrical neurological stimulus is applied at a frequency of from 30 to 45 GHz. In one embodiment, the electrical neurological stimulus is applied at a frequency of from 30 to 40 GHz.

In one embodiment, the electrical neurological stimulus is applied at a frequency of from 35 to 65 GHz. In one embodiment, the electrical neurological stimulus is applied at a frequency of from 40 to 65 GHz. In one embodiment, the electrical neurological stimulus is applied at a frequency of from 40 to 60 GHz.

In one embodiment, the electrical neurological stimulus is applied for a duration of from 1 to 60 minutes. In one embodiment, the electrical neurological stimulus is applied for a duration of from 5 to 60 minutes. In one embodiment, the electrical neurological stimulus is applied for a duration of from 10 to 60 minutes. In one embodiment, the electrical neurological stimulus is applied for a duration of from 15 to 60 minutes. In one embodiment, the electrical neurological stimulus is applied for a duration of from 20 to 60 minutes. In one embodiment, the electrical neurological stimulus is applied for a duration of from 25 to 60 minutes. In one embodiment, the electrical neurological stimulus is applied for a duration of from 30 to 60 minutes. In one embodiment, the electrical neurological stimulus is applied for a duration of from 35 to 60 minutes. In one embodiment, the electrical neurological stimulus is applied for a duration of from 40 to 60 minutes. In one embodiment, the electrical neurological stimulus is applied for a duration of from 45 to 60 minutes. In one embodiment, the electrical neurological stimulus is applied for a duration of from 50 to 60 minutes. In one embodiment, the electrical neurological stimulus is applied for a duration of from 55 to 60 minutes.

In one embodiment, the electrical neurological stimulus is applied for a duration of from 1 to 55 minutes. In one embodiment, the electrical neurological stimulus is applied for a duration of from 1 to 50 minutes. In one embodiment, the electrical neurological stimulus is applied for a duration of from 1 to 45 minutes. In one embodiment, the electrical neurological stimulus is applied for a duration of from 1 to 40 minutes. In one embodiment, the electrical neurological stimulus is applied for a duration of from 1 to 35 minutes. In one embodiment, the electrical neurological stimulus is applied for a duration of from 1 to 30 minutes. In one embodiment, the electrical neurological stimulus is applied for a duration of from 1 to 25 minutes. In one embodiment, the electrical neurological stimulus is applied for a duration of from 1 to 20 minutes. In one embodiment, the electrical neurological stimulus is applied for a duration of from 1 to 15 minutes. In one embodiment, the electrical neurological stimulus is applied for a duration of from 1 to 10 minutes. In one embodiment, the electrical neurological stimulus is applied for a duration of from 1 to 5 minutes.

In one embodiment, the electrical neurological stimulus is applied at a constant frequency over the duration of application.

In one embodiment, a period of time between the initial point of administration of the one or more cannabinoids and applying the electrical neurological stimulus is no greater than 120 minutes. In one embodiment, a period of time between the initial point of administration of the one or more cannabinoids and applying the electrical neurological stimulus is no greater than 90 minutes. In one embodiment, a period of time between the initial point of administration of the one or more cannabinoids and applying the electrical neurological stimulus is no greater than 60 minutes. In one embodiment, a period of time between the initial point of administration of the one or more cannabinoids and applying the electrical neurological stimulus is no greater than 55 minutes. In one embodiment, a period of time between the initial point of administration of the one or more cannabinoids and applying the electrical neurological stimulus is no greater than 50 minutes. In one embodiment, a period of time between the initial point of administration of the one or more cannabinoids and applying the electrical neurological stimulus is no greater than 45 minutes. In one embodiment, a period of time between the initial point of administration of the one or more cannabinoids and applying the electrical neurological stimulus is no greater than 40 minutes. In one embodiment, a period of time between the initial point of administration of the one or more cannabinoids and applying the electrical neurological stimulus is no greater than 35 minutes. In one embodiment, a period of time between the initial point of administration of the one or more cannabinoids and applying the electrical neurological stimulus is no greater than 30 minutes. In one embodiment, a period of time between the initial point of administration of the one or more cannabinoids and applying the electrical neurological stimulus is no greater than 25 minutes. In one embodiment, a period of time between the initial point of administration of the one or more cannabinoids and applying the electrical neurological stimulus is no greater than 20 minutes. In one embodiment, a period of time between the initial point of administration of the one or more cannabinoids and applying the electrical neurological stimulus is no greater than 15 minutes. In one embodiment, a period of time between the initial point of administration of the one or more cannabinoids and applying the electrical neurological stimulus is no greater than 10 minutes. In one embodiment, a period of time between the initial point of administration of the one or more cannabinoids and applying the electrical neurological stimulus is no greater than 5 minutes.

In one embodiment, the electrical neurological stimulus is applied using a bracelet or watch and the one or more cannabinoids are administered in edible form. In one embodiment, the electrical neurological stimulus is applied using a bracelet or watch and the one or more cannabinoids is a gummy.

In one embodiment, the one or more cannabinoids are administered twice a day as a gummy, once as a first dose and once as a second dose, wherein the first dose and second dose are independently administered in an amount of about 30 mg, and wherein the electrical neurological stimulus is applied twice a day, once as a first application and once as a second application.

In a second aspect, the present invention provides a non-therapeutic method of modulating a mood state of a subject comprising administering one or more cannabinoids in combination with the application of an electrical neurological stimulus to the subject, wherein the electrical neurological stimulus comprises millimetre wave technology.

In a third aspect, the invention provides a system comprising: one or more cannabinoids for delivery to a user in order to modulate a mood state of said user; and a delivery mechanism for applying an electrical neurological stimulus to a user in order to modulate a mood state of said user wherein the electrical neurological stimulus comprises millimetre wave technology.

In one embodiment, the system further comprises a control mechanism that controls the delivery of the one or more cannabinoids and the application of the electrical neurological stimulus to the user. For example, in one embodiment, the control mechanism is provided as part of an application on a mobile phone device or other electronic device.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future.

The invention will now be described with reference to the following non-limiting examples.

### Examples

### Example 1

138 non-clinical healthy participants took part in the study to assess the effect on the mood state of the participants after administration of a combination of CBD and millimetre wave technology.

The millimetre wave technology was administered using the wearable Remedee^{™} Labs millimetre wave emitting bracelet.

The CBD was administered as a gummy at a dose of 30 mg.

The participants were allocated to one of the four groups as shown in Table 1 below.

**Table 1**

| **Group 1** | **Group 2** | **Group 3** | **Group 4** |
|---|---|---|---|
| Active Remedee^{™} + CBD gummy | Active Remedee^{™} + Vitamin C gummy (CBD placebo) | Placebo Remedee^{™} + CBD gummy | Placebo Remedee^{™} + Vitamin C gummy (CBD placebo) |

The Placebo Remedee^{™} group were provided with an identically functioning device (i.e. lights showing that the device was switched on or off), wherein the device differed only in its ability to emit millimetre waves.

The participants underwent a 5 week active trial as outlined in Table 2 below.

**Table 2**

| | | Pre-study profiler survey |
|---|---|---|
| Week 1 | Each morning | Remedee^{™} device & gummy taken and morning survey completed within 30mins of usage. |
| | Each evening | Remedee^{™} device & gummy taken. Evening survey completed within 30mins of usage and at least 1 hour before sleep. |
| | End of week 1 | Weekly survey completed. |
| Week 2 | Each morning | Remedee^{™} device & gummy taken and morning survey completed within 30mins of usage. |
| | Each evening | Remedee^{™} device & gummy taken. Evening survey completed within 30mins of usage and at least 1 hour before sleep. |
| | End of week 2 | Weekly survey completed. |
| Week 3 | Each morning | Remedee^{™} device & gummy taken and morning survey completed within 30mins of usage. |
| | Each evening | Remedee^{™} device & gummy taken. Evening survey completed within 30mins of usage and at least 1 hour before sleep. |
| | End of week 3 | Weekly survey completed. |
| Week 4 | Each morning | Remedee^{™} device & gummy taken and morning survey completed within 30mins of usage. |
| | Each evening | Remedee^{™} device & gummy taken. Evening survey completed within 30mins of usage and at least 1 hour before sleep. |
| | End of week 4 | Weekly survey completed. |
| Week 5 | Each morning | Remedee^{™} device & gummy taken and morning survey completed within 30mins of usage. |
| | Each evening | Remedee^{™} device & gummy taken. Evening survey completed within 30mins of usage and at least 1 hour before sleep. |
| | End of week 5 | Weekly survey completed. |
| | | Exit Survey |

### Measurement Methods

In order to assess the mood state of the participants, a number of daily and weekly surveys were used. These are outlined below:

### Instruments in Profiler Surveys

POMS 35-scale short form (Profile of Mood States) survey
STAI-T 10-scale short form (The State-Trait Anxiety Inventory - Trait portion only) survey QOLS (The Quality of Life Scale)
PROMIS Sleep Disturbance (Patient-Reported Outcomes Measurement Information System) survey
PROMIS Sleep Impairment (Patient-Reported Outcomes Measurement Information System) RSQ-W (Restorative Sleep Questionnaire)
Athens Insomnia Scale (AIS)

### Daily Surveys

VAMS (Visual Analogue Mood Scales) survey (am and pm)
STAI-S 6-scale short form (The State-Trait Anxiety Inventory - State portion only) survey (am and pm)

### Weekly Surveys

POMS 35-scale short form (Profile of Mood States) survey
PROMIS Sleep Disturbance (Patient-Reported Outcomes Measurement Information System) survey
PROMIS Sleep Impairment (Patient-Reported Outcomes Measurement Information System) survey
RSQ-W (Restorative sleep Questionnaire)

### Exit Survey

QOLS (The Quality of Life Scale)
Athens Insomnia Scale (AIS)

### Results

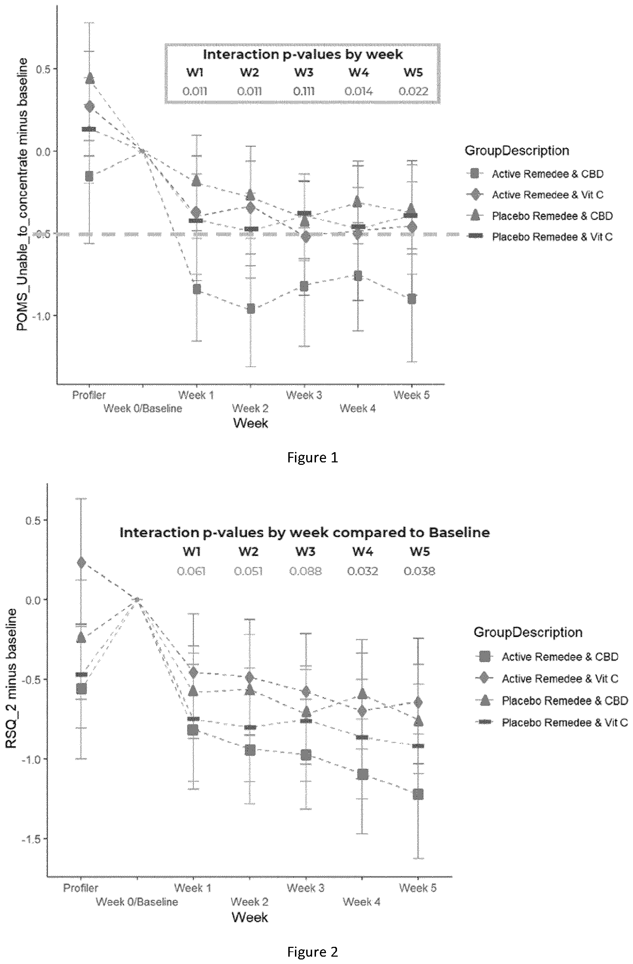

When both the active Remedee^{™} device and GBD gummy are taken/used together (Group 1), it was found that there was a larger impact on the participant's ability to concentrate when compared to Groups 2, 3 and 4. In particular, as shown in Figure 1, a significant interaction was seen in 4 out of the 5 weeks.

In addition, when both the active Remedee^{™} and CBD gummy are take/used together (Group 1), there is a larger impact on the participant's perceived sleepiness when compared to Groups 2, 3 and 4. In particular, as shown in Figure 2, a significant interaction was seen in 3 out of the 5 weeks.

These results show that the combination of millimetre wave technology and one or more cannabinoids has a positive impact on a user's ability to concentrate and reduction in a user's perceived sleepiness. This demonstrates that the administration of one or more cannabinoids in combination with the application of an electrical neurological stimulus to the subject, wherein the electrical neurological stimulus is millimetre wave technology can allow for improved modulation of a mood state of the subject.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future.

## Claims

1. A method of modulating a mood state of a subject comprising administering one or more cannabinoids in combination with the application of an electrical neurological stimulus to the subject, wherein the electrical neurological stimulus is millimetre wave technology.

2. The method of claim 1, wherein the one or more cannabinoids are inhaled.

3. The method of any one of claims 1 or 2, wherein the one or more cannabinoids are administered by an aerosol delivery device.

4. The method of claim 1, wherein the one or more cannabinoids are ingested.

5. The method of claim 4, wherein the one or more cannabinoids are ingested orally.

6. The method of claim 5, wherein the one or more cannabinoids are in edible form.

7. The method of any one of claims 1 to 6, wherein the one or more cannabinoids are administered at a total daily dose of no greater than 200 mg.

8. The method of any one of claims 1 to 7, wherein the one or more cannabinoids are administered twice a day, once as a first dose and once as a second dose, wherein the first dose and the second dose are each independently administered in an amount from 5 and 50 mg.

9. The method of any one or claims 1 to 8, wherein the one or more cannabinoids have an initial point of administration that is concurrent with, before, or after the application of the electrical neurological stimulus.

10. The method of any one of claims 1 to 9, wherein a period of time between the initial point of administration of the one or more cannabinoids and applying the electrical neurological stimulus is no greater than 60 minutes.

11. The method of any one of claims 1 to 10, wherein the electrical neurological stimulus is applied by an implant.

12. The method of any one of claims 1 to 10, wherein the electrical neurological stimulus is applied by a wearable device.

13. The method of claim 12, wherein the wearable device is a bracelet, a watch, a headband, or chestband.

14. The method of any one of claims 12 or 13, wherein the wearable device is a bracelet.

15. The method of any one of claims 1 to 14, wherein the electrical neurological stimulus is applied twice a day.

16. The methods of any one of claims 1 to 15, wherein the one or more cannabinoids are administered twice a day, once as a first dose and once as a second dose and the electrical neurological stimulus is applied twice a day, once as a first application and once as a second application.

17. The method of any one of claims 1 to 16, wherein the mood state comprises the subject's ability to concentrate.

18. The method of any one of claims 1 to 17, wherein the mood state comprises the subject's perceived sleepiness.

19. A non-therapeutic method of modulating a mood state of a subject comprising administering one or more cannabinoids in combination with the application of an electrical neurological stimulus to the subject, wherein the electrical neurological stimulus is millimetre wave technology.

20. A system comprising: one or more cannabinoids for delivery to a user in order to modulate a mood state of said user; and a delivery mechanism for applying an electrical neurological stimulus to a user in order to modulate a mood state of said user, wherein the electrical neurological stimulus is millimetre wave technology.

21. The system of claim 20, further comprising a control mechanism that controls the delivery of the one or more cannabinoids and the application of the electrical neurological stimulus to the user.
